# EUROPEAN PATENT SPECIFICATION

(11) **EP 1 863 922 B1**
(45) Date of publication and mention of the grant of the patent: **27.02.2013**
(21) Application number: 05714732.4
(22) Date of filing: 01.04.2005
(51) Int. Cl.: C12Q 1/68

(54) **PRIMERS FOR AMPLIFICATION AND SEQUENCING OF EUBACTERIAL 16S rDNA FOR IDENTIFICATION**
PRIMER ZUR AMPLIFIKATION UND SEQUENZIERUNG EUBAKTERIELLER 16S-RDNA ZUR IDENTIFIZIERUNG
AMORCES POUR L'AMPLIFICATION ET LE SEQUENÇAGE D'ADNR 16S EUBACTERIEN EN VUE DE L'IDENTIFICATION

(43) Date of publication of application: 12.12.2007
(73) Proprietor: Smartgene GmbH, 6300 Zug (CH)
(72) Inventor: EMLER, Stefan, CH-8032 Zürich (CH)
(74) Representative: Müller-Boré & Partner Patentanwälte
(86) International application number: PCT/CH2005/000190
(87) International publication number: WO 2006/102772

(56) References cited:
- WO-A-90/15157
- WO-A-99/58713
- WO-A-2004/044247
- WO-A-2004/060278
- US-A1- 2003 082 535
- GREISEN ET AL: "PCR probes and primers for the 16S rRNA of most species of pathogenic bacteria found in Cerebrospinal fluid" JOURNAL OF CLINICAL MICROBIOLOGY, WASHINGTON, DC, US, vol. 32, no. 2, February 1994 (1994-02), pages 335-351, XP002080618 ISSN: 0095-1137
- OKHRAVI N ET AL: "PCR-RFLP-mediated detection and speciation of bacterial species causing endophthalmitis" INVESTIGATIVE OPHTHALMOLOGY & VISUAL SCIENCE, ASSOCIATION FOR RESEARCH IN VISION AND, US, vol. 41, no. 6, May 2000 (2000-05), pages 1438-1477, XP002985596 ISSN: 0146-0404

### SEQUENCE LISTING

<110> SmartGene GmbH
<120> Primer für 16S rRNA
<130> F01696
<160> 2
<170> PatentIn version 3.1
<210> 1
<211> 19
   <212> DNA
   <213> artificial sequence
<220>
   <223> primer
<400> 1
   cgctcgttgc gggacttaa 19
<210> 2
   <211> 21
   <212> DNA
   <213> artificial sequence
<220>
   <223> Primer
   <220>
<221> misc_feature
   <222> (1) .. (21)
   <223> n = Inosine
<400> 2
   gagagtttga tcntggctca g 21

## Description

### TECHNICAL FIELD

The invention relates to oligonucleotides and their use for the qualitative and/or quantitative amplification and/or the sequencing of 16S rDNA-genes, as well as of fragments thereof and RNA derived thereof. It in particular relates to the identification of bacteria.

### BACKGROUND OF THE INVENTION

Bacterial contamination of food, water, or soil can result in serious illness in humans and animals. Detection and identification of pathogenic organisms are important for containment of potential epidemics, for elimination of natural host reservoirs, for prevention of further contamination, and for appropriate subsequent treatment should exposure occur. Rapid detection and identification of the sources of contamination provides the information to properly eliminate and prevent the spread of bacteria so as to ensure the quality and safety of food and water resources, and the prevention of epidemics.

The same applies if a human or animal has been infected by bacteria, also here the rapid detection and specific identification of the bacterial species or strain from clinical specimens is key to subsequent treatment.

For the identification of bacteria in various media classically cultivation of the bacteria is used for amplification and subsequent depiction/identification. However, cultivation of the bacteria is time-consuming and in particular, in many cases neither the species, and even less the strain can be safely and unambiguously determined.

The polymerase chain reaction (PCR) is an in-vitro method of amplifying DNA sequences. Target DNA from a bacterial source of interest may be amplified and detected in minute quantities. The target DNA to be amplified must be flanked by a known sequence of several nucleotides; short pieces of DNA are synthesized with sequences identical to the known sequences; these are referred to as oligonucleotide primers or simply primers. The PCR process usually requires denaturing the target DNA strand into two separate strands by heating it to 90-98°C. A predetermined primer is then annealed to each of the separate strands at the flanking positions under hybridisation conditions. A heat-resistant enzyme, referred to as Taq polymerase, synthesizes a strand of DNA complementary to the existing DNA strands to form two complete double DNA copies of the original starting target DNA. By repeating this process once, four double-stranded chains are formed. Every time the process is repeated, the amount of PCR product is exponentially increased.

The amplified PCR product can subsequently be used, i.e. subjected to specific marker systems, for example specific sequences which hybridise with genes of specific bacterial strains, in order to identify the strain.

The following documents can be mentioned disclosing primers for 16S and/or 23S rRNA in the general context of the present invention:

US 2003082535 discloses the evaluation of a sample for the presence and qualitative nature of a microorganism can in a single vessel by combining a natural abundance DNA sample with a sequencing mixture containing a primer, a thermally stable polymerase such as ThermoSequenase which incorporates dideoxynucleotides into an extending nucleic acid polymer, nucleotide triphosphate feedstocks, and a chain terminating nucleotide triphosphate. The mixture is processed through multiple thermal cycles for annealing, extension and denaturation to produce a product mixture which is analyzed by electrophoresis.

WO 9958713 relates to a detection method and a test kit for economic detection of germs in pharmaceutical and cosmetic products. The invention uses specific probes and primers whose replication is made visible by means of a special indicator system, whereby a fluorescent colorant is released.

The publication "PCR primers and probes for the 16S rRNA gene of most species of pathogenic bacteria, including bacteria found in cerebrospinal fluid (Greisen et al, J. Clin. Microbiol, 32, 2, p335-351) tests and discloses a broad range of PCR primers for the 16S rRNA gene in bacteria, however for each class of bacteria different primers are proposed.

WO 90/15157 discloses nucleic acid probes capable of hybridizing to rRNA of eubacteria and not to rRNA of non-eubacteria along with methods utilizing such probes for the detection of eubacteria in clinical and other samples. Preferred embodiments include probes capable of distinguishing between gram-positive and gram-negative bacteria.

WO 2004060278 provides methods of identifying pathogens in biological samples from humans and animals, resolving a plurality of etiologic agents present in samples obtained from humans and animals, determining detailed genetic information about such pathogens or etiologic agents, and rapid detection and identification of bioagents from environmental, clinical or other samples. Also here a number of possible primers is disclosed.

WO 2004044247 relates to a novel sample preparation, probes, couple primer sets for one step real time reverse transcriptase polymerise chain reaction (RT-PCR), methods and kits for the universal detection of alive bacteria and/or fungus-yeast in pharmaceutical, cosmetic and non clinical samples.

### SUMMARY OF THE INVENTION

The objective problem underlying the present invention is therefore to provide improved systems of oligonucleotides for the identification of the bacterial genera/species/strains present in particular in a clinical specimen or in bacterial cultures, as well as methods for using these oligonucleotides for the identification. In particular the invention relates to oligonucleotides for the qualitative and/or quantitative amplification and/or the sequencing of 16S rDNA-genes, as well as of fragments thereof and RNA derived thereof.

The present invention solves the above problem in that primers are being used which are only hybridizing with regions of the 16S rDNA-gene which are highly conserved. The primers are aiming at binding to these highly conserved regions and when using the PCR amplification at generating amplicons which comprise strain specific regions between the conserved regions to which the primers are binding such that for example using sequencing of the full amplicons these strain specific regions can be analysed and correspondingly the bacterial system identified unambiguously.

So the primers aiming at highly conserved regions of the 16S rDNA or 16S rRNA allow broadband PCR amplification, i.e. amplification which is substantially independent of the genera/species/strains of the bacterial cultures to be identified. On the other hand these highly conserved regions of the 16S rDNA or 16S rRNA enclose highly strain-specific regions, which will be reflected in the generated amplicons. Sequence analysis of the amplicons correspondingly allows identification of the genera/species/strains of the bacterial cultures based on the sequence of these highly strain-specific regions which are present in the fragment. As a matter of fact, the proposed primers have been tested with more than 800 clinical isolates of more than 80 genera and more than 190 different species, and are surprisingly working for this vast group of bacterial systems proving that their functioning is substantially independent from the bacterial genus, allowing broadband detection/amplification etc.. The primers may be used for amplification, sequencing, marking, as probes etc.

The proposed identification of bacteria by 16S rDNA sequence analysis allows identification of cultures within 24h, regardless of growth or metabolism and can identify fastidious and novel microorganisms.

The object of the present invention are therefore pairs of oligonucleotides selected from an oligonucleotide consisting of the sequence called BR16SR (SEQ ID No. 1 as given in the attached sequence listing):
CGCTCGTTGC GGGACTTAA (5'-3'; 19mer; reverse)
   and an oligonucleotide consisting the sequence B162 (SEQ ID No. 2 as given in the attached sequence listing):
GAGAGTTTGA TCNTGGCTCA G (5'-3'; 21mer; forward)
wherein N stands for the placeholder Inosine.

Preferably, the selective hybridisation takes place under stringent conditions only, wherein for example under PCR using recombinant DNA polymerase from Thermus aquaticus the stringent conditions are defined as: 50-60°C, at 500 nM MgCl₂.

According to the present invention, surprisingly two highly conserved regions could be found which embrace a strain specific strand of approximately 1000-1200 bases, and which are located at the positions (-3) to 18 and 1098 to 1080 as defined in *E.Coli* X80724 (using a different reference system, the positions may change). Correspondingly, according to the present invention, preferably the oligonucleotide comprising the sequence SEQ ID No. 1 under stringent conditions specifically hybridises with the specific region from 1098 to 1080 as defined in *E.Coli* X80724, and the oligonucleotide comprising the sequence SEQ ID No. 2 under stringent conditions specifically hybridises with the specific region from (-3) to 18 as defined in *E.Coli* X80724. Since the positioning depends on the reference system, also shiftings by no more than 10, preferentially shiftings by not more than 4 nucleotides in respect to these specific regions should be taken account of.

The oligonucleotides may comprise at least one marker and/or they may be attached to a matrix for a specific purposes.

For amplification purposes using PCR, the above-mentioned oligonucleotides according to sequences SEQ ID No. 1 and SEQ ID No. 2 are used as a pair in order to generate the desired fragment which is bordered by the two primers. Preferentially, such a mixture of the two primers comprises these oligonucleotides (and/or the complementary oligonucleotides) in equal amounts.

Furthermore, the present invention also relates to a method for the specific amplification and/or the sequencing of 16S rDNA-genes, as well as of fragments thereof and RNA derived thereof, wherein as primer a mixture as given above are used.

In case of amplification of the 16S rDNA-gene, or the fragments thereof or the RNA derived thereof the oligonucleotides are brought into contact with a mixture of the pair of the distinct oligonucleotides SEQ ID No. 1 and SEQ ID No. 2 (and/or the complementary oligonucleotides) acting as reverse and forward primer, respectively, and are subjected to a polymerase chain reaction leading to specific marker fragments with in the range of 100-2000, preferentially in the range of 800-1200 nucleotides. Contact between the 16S rDNA-gene, or the fragments thereof or the RNA derived thereof, with a mixture of the pair of the distinct oligonucleotides SEQ ID No. 1 and SEQ ID No. 2 (and/or the complementary oligonucleotides) is preferably established under stringent conditions, which in case of PCR using recombinant DNA polymerase from Thermus aquaticus the stringent conditions are defined as: 55°C, 500 nM MgCl₂.

Typically, in a first step the 16S rDNA-gene, or the fragments thereof or the RNA derived thereof is heat-denaturated in order to obtain single-stranded chains, subsequently these are brought into contact with a mixture of the pair of the distinct oligonucleotides SEQ ID No. 1 and SEQ ID No. 2 (and/or the complementary oligonucleotides), and then extended by means of a DNA polymerase, and wherein the heat-denaturation and the extension cycles are repeated in order to obtain a detectable amount of product.

Furthermore, the present invention relates to the use of the above-mentioned oligonucleotides for sequencing purposes. Correspondingly, a method is proposed for the sequencing using the chain termination method (or Sanger method), in which a sample is sequenced in both directions using either the reverse, SEQ ID No. 1, or the forward, SEQ ID No. 2 (and/or the complementary oligonucleotides), as a primer. Also here, preferably for the amplification steps in the sequencing reaction stringent conditions are used, which e.g. means that under PCR using recombinant DNA polymerase from Thermus aquaticus the stringent conditions are defined as: 52°C, 500 nM MgCl₂.

In addition to that, presently a method is proposed for the identification of bacterial species based on their 16S rDNA-gene. This method uses the following steps a) sample material of a bacterial colony is isolated and the genetic material is extracted or at least made accessible to amplification; b) the thus derived material is subjected to amplification in accordance with the method as described above; c) the double-stranded DNA amplification products are purified; d) the purified product is subjected to cycle-sequencing using a method as described above, preferably with the chain termination principle (for the sequencing however in step d), also completely different sequencing methods may be used); e) the sequenced sample is purified; f) the purified and sequenced sample is subjected to sequence electrophoresis and signal recording. Preferably, for the initial amplification in step b) as well as for the sequencing in step d) the same set of primers (and/or the complementary oligonucleotides) as given above is used, i.e. for the initial amplification the pair of primers is concomitantly used for generating the fragment of preferably approximately 1030bp, and in the two sequencing reactions (from both sides) either of the two primers is used.

For the identification of the bacterial genus/species/strain present in the bacterial colony the raw sequence data as obtained in step f) are preferably automatically aligned, noise sequences are optionally stripped, a consensus sequence is created by comparing the measured data with sequence data from references from a database of known bacterial genera/species/strains, all this using computer implemented methods.

Further embodiments of the present invention are outlined in the dependent claims.

### SHORT DESCRIPTION OF THE FIGURES

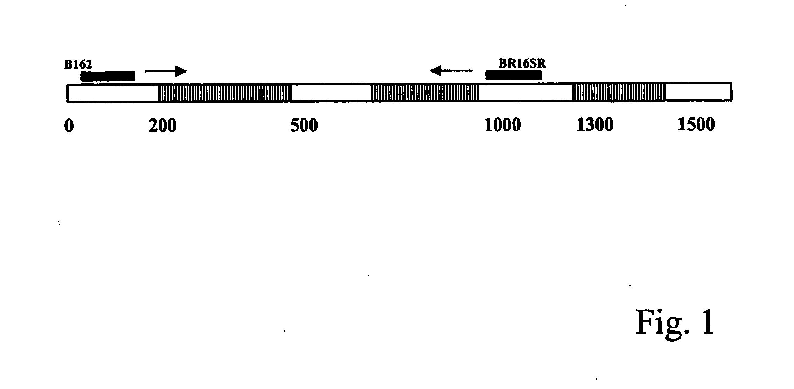

In the accompanying Figure 1, the fragment of 16S rDNA/rRNA as chosen for analysis is displayed schematically with variable and conserved regions as well as the positions of the primers are indicated.

### DETAILED DESCRIPTION OF THE PREFERRED EMBODIMENTS

The gene for the 16S subunit of the bacterial ribosome is highly conserved over several stretches in all eubacteria. Using the specific primers according to the present invention, which are named B162 (SEQ ID No. 2) and BR16SR (SEQ ID No. 1) to conserved regions in all known bacteria, a broad-range PCR allows amplification of a >1000 bp fragment of the 16S rDNA starting with crude DNA-preparations out of any suitable bacterial culture.

In this respect see figure 1 which displays this stretch of the gene, and in which hatched regions indicate possible strain-specific regions (their position may vary) and in which white areas show the conserved parts.

For a secure identification of the bacterial isolate amplified, the same primers are preferentially as used for amplification of the genetic material are applied to direct sequencing of the amplified product. e.g. using the Sanger method or equivalent methods which rely on the use of a primer. Any commercially available direct-sequencing technology or DNA sequencer is suitable for this purpose.

Since the positions of the so-called "species-specific variable regions" (hatched in Figure 1) vary between the different bacterial genus, it is important that the amplification primers span a region large enough to contain at least one or two variable regions within the fragment with sufficient differentiation with regard to a to-be-identified bacterial isolate. The fragment of about 1030 bp presently opted for demonstrably contains this variability for the differentiation of almost all clinically relevant bacterial species.

Technically, a fragment of 1000 bp can be sequenced from both sides with only two sequencing reactions (using the amplification primers) and therefore saves costs, time and material while preserving high analytical accuracy; with ongoing progress in sequencing technology it is conceivable that only one sequencing reaction may be applied in the future.

A vast range of different genera (more than 80) and corresponding species (more than 190) has been tested on more than 800 clinical isolates to be identifiable with the proposed primers. A list is given further below.

The primers B162 (SEQ ID No. 2) and BR16SR (SEQ ID No. 1) are both designed to hybridize specifically to conserved parts of the bacterial 16S rRNA gene (16S rDNA) and to produce a fragment of about 1030 bp, including variable regions independently of their position within this fragment. In order to avoid misalignment, one primer B162 (SEQ ID No. 2) includes a non-specific placeholder N (Inosin) instead of a A, C, G or T.

In Table 1 the sequences of the two primers are given as well as their positioning within the 16S rDNA gene of *Escherichia coli* NCBI X80724, and their annealing temperatures as calculated using the Wallace formula (Tₘ = 2 (A + T) + 4 (G + C)), wherein Inosine is counted as T/A.

**Table 1**

| Oligonucleotide | SEQ ID | Sequence¹ | Position² | Tm |
|---|---|---|---|---|
| BR16SR | No. 1 | cgctcgttgc gggacttaa | 1098-1080 | 60° |
| B162 | No. 2 | gagagtttga tcntggctca g | (-3) to 18 | 60° |

| | | | | |
|---|---|---|---|---|
| ¹: n = Inosine ²: Position in the 16S rDNA gene of *Escherichia coli* NCBI X80724. | | | | |

For a secure identification of the sequencing data, only material from one strain/colony should be used; mixed cultures and contaminated primary material may result in "unreadable" overlay sequences and should be avoided. See below for details on the preparation of different samples. If other technologies than direct sequencing are applied to differentiate the variable species-specific regions (e.g. micro-array, hybridization, cloning and sequencing...), this restriction to single (pure) colonies does not apply.

In the following, the specific procedure for amplification and subsequent sequencing for identification of eubacterial 16S rDNA is given, however, this specific example should not be used to limit the scope of the invention as claimed in the appended claims. In particular variants thereof which are within the scope of the general knowledge and practice of the person skilled in the art are explicitly included.

### 1. Testmaterial / Software

Kit: Big-Dye Terminator Cyle Sequencing (Applied Biosystems Art.-Nr. 4303152)
Kit: QIAamp DNA mini kit (Qiagen Art.-Nr. 51306)
Kit: QIAquick PCR purification kit (Qiagen Art.-Nr. 28106)
Kit: DyeEx spin kit (Qiagen Art.-Nr. 63106)
Heating-block 95-96°C
Ultrasonic unit (e.g. Abbott ,,LCX Lysor")
Benchtop centrifuge (e.g. Eppendorf 5415D)
Thermocycler (e.g. PE 9600)
Equipment for gel-electrophoresis, including gel-trays with casting-facility, power supply, staining/destaining trays, transilluminator, Polaroid-camera and specific reagents
Speedvac (e.g. Eppendorf Concentrator 5301)
Sequencing-device (e.g. ABI / PE310) including peripheral equipment and reagents (long capillary, tubes, caps, polymer = POP-6, seq. buffer 10x, template suppression reagent = TSR)
Tubes 1.5ml (Eppendorf and Sarstedt) and 0.2ml (Sarstedt multiply pro) width tubeholders
H₂O HPLC grade, sterile; TE pH 8.0 / 7.5 (10 mM Tris-Cl, 1 mM EDTA); 0.9% NaCl sterile
Glass beads, acid washed (Sigma G-4649),
Ampli-Taq LD (Applied Biosystems Art.-Nr. N808-0107)
Primers for eubacterial 16s rRNA broad-range PCR, specifically B162 (SEQ ID No. 2) and BR16SR (SEQ ID No. 1).
Software: 310 Collection; Sequencing Analysis; MT Navigator PPC, IDNS™ by SmartGene, with account

### 2. Sample material

1 loop of a bacterial colony from a culture dish, suspended in TE pH 8.0 or out of liquid culture; avoid agar.

Primary material can be used in certain cases (i.e. when collected under sterile conditions, as for CSF or joint-punctuations). Contaminated starting material such as sputum results in multiple, unreadable overlaid signal peaks.

### 3. Preparation of Reagents:

Use sterile filtertips on all clinical samples/cultures/DNA-preparations in order to avoid contamination. Label all reagent-tubes. All clinical material has to be considered as potentially infectious and should be handled in laminar flow benches only unless inactivated at 95°C for at least 15 min.

### 3.1. General preparations

Extraction: preheat heating-block to 96°C. Amplification: pre-heat the thermocycler, pour a 2% agarose-gel and let it cool down for at least 2h. Cycle-sequencing: pre-heat the thermocycler. Sequencing: switch on speedvac (30°C), equilibrate Template Suppression Reagent (TSR) and 1x Seq.-Puffer to RT , preheat heating-block to 96°C, switch on DNA-Sequencer and computer.

### 3.2. Sample preparation

Suitable bacterial colonies on solid medium are transferred without any culture medium (possible inhibition of PCR) from the culture to a 1.5 ml Sarstedt screw-cap tube, previously filled with 300ml TE, mix gently. Bacterial suspensions can be stored at 4°C for a maximum of 5 d.

Liquid-cultures: transfer approx. 1 ml of bacterial suspension to a 1.5ml Sarstedt tube and centrifuge for 5 min. at 5000 rpm. Discard the supernatant in the laminar-flow bench using filter-tips and resuspend the pellet in 500ml 0.9% NaCl. Spin down, discard supernatant and resuspend the pellet in 300ml TE, then follow standard-procedure.

Primary specimens, like whole blood, CSF (CerebroSpinal Fluid), liquid from pleural-punctures may be used only if it can be assumed that just one predominant germ is present and contamination by flora can be excluded. Material should be treated similarly to the liquid-culture procedure.

### 4. Extraction

Use the QIAamp DNA mini kit for the extraction of clinical specimens or for culture suspensions containing inhibitory substances such as agar etc.
1. Gently mix the bacterial suspension in the closed screw-cap Sarsted tubes and incubate them for 15 min. at 96°C in order to inactivate the bacteria. Cool down for 5 min. at RT, mix and quick-spin, in order to avoid lid contamination.
2. Add approx. 100ml glass-beads (Sigma) by using a 1000ml pipette with filter tip (fixed to 5.0ml, "dry" pipetting). Close caps and vortex briefly.
3. Place all sample tubes symmetrically on the dry sonicator Lysor-plate, equilibrate with mock-tubes if necessary. Sonicate for approx. 15 min., when finished, remove tubes, vortex and spin down the glass-beads (5 min. at 13'000rpm).
4. Immediately transfer the supernatants into new 1.5 ml Eppendorf tubes in order to avoid adsorption of DNA to glass-beads.
5. Qiagen-extraction: samples out of liquid-cultures and potentially inhibited samples should be further purified using silica-columns. For each sample use 200ml of supernatant post sonication and follow the Qiagen protocol (QIAamp DNA mini kit).

### 5. Amplification

Standard PCR-master-mix can be prepared previously, including the LD-AmpliTaq.
1. Instruction for master-mix: See Table 2 for a typical pipetting setup of single components useful for one test.

**Table 2: Master-mix-setup for broad-range PCR using standard primers**

| components | conc. stock sol. | | volume (one test) | volume | final conc. | |
|---|---|---|---|---|---|---|
| H₂O HPLC-grade | --- | | 58.77 | µl | --- | |
| reaction buffer (PE) | 10.00 | x | 10.00 | µl | 1.00 | x |
| MgCl₂ (1) | 150.00 | mM | 0.33 | µl | 500.00 | nM |
| dNTP | 2.00 | mM | 25.00 | µl | 500.00 | µM |
| primer B 162 (2) | 50 | µM | 0.20 | µl | 100.00 | nM |
| primer BR16SR (2) | 50 | µM | 0.20 | µl | 100.00 | nM |
| AmpliTaq LD (PE) | 5.00 | U/µl | 0.50 | µl | 2.50 | U/rx |
| Totalvol. without template | | | 95.00 | µl | | |

| | | | | | | |
|---|---|---|---|---|---|---|
| (1) including. 1,5 mM already contained in the reaction buffer; (2) concentration per ml TE pH 7.5 | | | | | | |

2. The corresponding broad-range PCR for mycobacteria (master-mix EU2) with B162 and BR16SR is thawed, mixed briefly, centrifuged and pipetted in aliquots of 95ml into labelled 0.2ml PCR single-tubes (Sarstedt Multiply pro). Include one negative, one positive control to each amplification; potentially inhibited samples or primary clinical specimens may require a spiked inhibition control or a dilution 1:5.
3. For the negative control, add 5ml of H₂O (HPLC-grade) to the master-mix.
4. For the positive amplification-control or for inhibition control, add 5 µl of previously successfully amplified DNA to the master-mix.
5. Add 5ml of each sample DNA-suspension to the master-mix, close caps immediately after pipetting in order to avoid cross contamination.
6. Amplification on a thermocycler (ex. PE 9600): 3' 95°C, 38x [30" 95°C, 30" 55°C, 45" 72°C], 5' 72°C, 99h 5°C.
7. After PCR, caps of PCR tubes are opened one by one and 10ml of each amplification product is analyzed on a 2% agarose-gel, stained with ethidium-bromid and visualized on a transilluminator. Only samples with clear bands of the proper size should be used for further processing.

### 6. Purification after amplification

Use the QIAquick PCR purification kit for direct purification of double-stranded (ds) DNA PCR amplification products. This procedure is mainly the manufacturer's recommendation for purification of PCR products for sequencing purposes.
1. Spin down all PCR-amplicons in 0.2 ml single tubes (cf 10" at ≥ 10`000 x g).
2. Pipet 500ml (5 Vol.) of PB buffer (Qiagen) for each sample into a clean, labelled 1.5ml reaction tube, then add the PCR-amplicons (approx. 90ml) using filtertips. Mix briefly on a vortex. For 50 ml PCR volume, adjust PB buffer accordingly.
3. Label the QIAquick columns and put them in the collection tubes provided with the kit
4. Quick-spin all samples; transfer liquid on the corresponding QIAquick spin column (use filter-tips); amplicons will bind to the silica-matrix.
5. Spin for 60 sec. at ≥10'000 x g (approx. 13'000rpm on a Eppendorf centrifuge 5415D).
6. Transfer the QIAquick column to a fresh collection tube, discard the eluate.
7. Wash the QIAquick columns by adding 750ml PE buffer (Qiagen, must be diluted in advance with 99% ethanol HPLC according to instructions) and spin for 60 sec. at ≥ 10'000 x g
8. Again, transfer the QIAquick column to a fresh collection tube and discard the eluate. Spin for 60 sec. at ≥10'000 x g. This additional centrifugation step is needed for complete removal of any residual ethanol.
9. Transfer the QIAquick column to a fresh, labelled 1.5 ml reaction tube and discard the eluate.
10. To elute the amplicon, pipet 50ml EB Puffer (Qiagen, 10mM Tris, pH 8.5) directly to the center of the QIAquick membrane. In order to increase the concentration of amplicons, use only 30ml EB and incubate at RT for 1 min. prior to centrifugation for 60 sec. at ≥10'000 x g. Remove column and store purified PCR products up to 7d at +4°C, or at - 20°C for longer periods.

### 7. Cycle-Sequencing

The AB DNA Big Dye Terminator Sequencing Kit can be used to sequence and end-label purified broad-range PCR products with BigDye-fluorescence markers. Same primers, i.e. B162 and BR16SR, as for PCR are used in order to sequence the entire amplicon, to use one primer per reaction.
1. Each sample is sequenced in both directions using forward (B162) and reverse primers (BR16SR). As positive control for the sequencing reaction, the plasmid provided with the sequencing kit can be used, together with the kit-primer.
2. The following components are pipetted into 0.2ml PCR tubes (Table 3): H₂O HPLC grade, ad 20ml, 4ml Cycle-Seq master-mix, 1ml primer B162 or BR16SR (10 µM) and 1-10ml purified broad-range PCR product (depending on the signal on the gel). Double volume of the mastermix to 8ml when sequencing samples with faint signals on gel.

| No. tub | Pos. Seq. | Gel: slot | dir. (f/r) | Vol. [µl] | primer | Vol. [µl] | MMx [µl] | H₂O [µl] | total [µl] |
|---|---|---|---|---|---|---|---|---|---|
| 2 | A3 | xx | f | 1 | B162 | 1 | 4 | 14 | 20 |
| 3 | A5 | xx | r | 1 | BR16sr | 1 | 4 | 14 | 20 |
| 4 | A7 | yy | f | 5 | B162 | 1 | 8 | 6 | 20 |

Table 3: Pipetting-scheme for the setup of a BigDye Terminator Cycle sequencing reaction. ,,Pos.Seq" relies on the position of the sample on the 48-sequencing-tray of the PE310.
3. Amplification on a thermocycler: 1' 96°C, 25x [10" 96°C, 5" 52°C, 4' 60°C], 99h 4°C. The Cycle-sequencing amplification products may be stored up to 5d at 4°C, protected from light.

### 8. Purification of the sequenced sample

The QIAGEN DyeEx Spin Kit is used to remove unincorporated Big-dye ddNTP from the sequencing reaction.
1. Carefully spin down sample tubes (60 sec. at ≥ 10'000 x g).
2. For each sample to be purified label a gel-column (red cap), invert briefly, flip of lower closure and loosen cap a quarter of a turn.
3. Centrifuge the columns for 3 min. at 750 x g (3000rpm on a Eppendorf centrifuge 5415D) and transfer them into 1.5ml reaction tubes.
4. Carefully pipet the sequencing-reaction-mixtures (10-20ml) on the corresponding columns, avoiding to touch neither the column-walls nor the gel-slurry. Samples with a volume lower than 10ml have should be expanded to 20ml with H₂O HPLC grade.
5. Centrifuge the columns (cf 3 min. at 750 x g), the eluate contains the sequencing-sample. Discard the column.
6. Dry all samples for 30 min. at 30°C in a speedvac (caps toward center). Dried samples are stable indefinitely at 4°C, light-protected.

### 9. Sequence electrophoresis and signal recording

A capillary-sequencer such as the ABI Prism310 Genetic Analyzer can be used.
1. Rinse capillary first, use yellow tape to fix it approx. 0.5mm below the anode, then switch on sequencer and computer. The apparatus initializes and homes automatically syringe and autosampler. Calibration of the capillary is required only after replacement of the anode or the capillary. Set capillary to position 3 (H₂O, "Autosampler To Position", then "Autosampler Up" approx. 500 steps until end dips in completely).
2. The lyophilized samples from the cycle sequencing reaction are resuspended in 25ml TSR (Template-Suspension Reagent), mixed well and briefly centrifuged (quick-spin).
3. Denature all samples for 2 min. at 95°C and chill down immediately on a cooler. Mix well and quick-spin. The samples are then transferred into labelled sequencing tubes using filter tips, closed with rubber stoppers and placed onto the 48-rack at the same positions as indicated in the injection list. The rack is fixed onto the autosampler and both doors are closed after a second push on "TRAY".
4. Sample Sheet (File/New ,,Sequence Smpl Sheet 48 Tube"): position A1 is always CCD (prerun testing of the photo-detectors), then all samples according to the injection list. Choose the Dye Set/Primer ,,DT POP6 {BD Set-any Primer}" and Matrix "Seq Matrix E".
5. Injection List: Length to Detector = 50 cm (long capillary). Controls: CCD-Test (set module to "CCD pre-run") always at the beginning, pGEM -if needed- at the end of a sequencing run.
6. Start the sequencing. CCD-test should result in signal curves below 2000, if the values are too high capillary and laser-window have to be cleaned carefully using H₂O and 70% EtOH.
7. Additional samples can be inserted during the run if desired.
8. Place the new tubes on the rack of the autosampler and close doors to resume the run automatically.
9. Once the run is finished, remove all tubes from the rack and store at 4°C until successful analysis of the raw data is finished. Park capillary at position 3 (water) and move it down until submerged. Avoid drying out of the capillary.

### 10. Analysis of the sequencing data with the ProofReader-IDNS(TM)

Sequencer manufacturer software can be used for analysis. Preferably the software package ProofReader-IDNS™ as available from the applicant is used as it allows the validation of raw data by comparing them to target-specific references. Moreover, this software package allows:
• Direct upload of raw sequence data (electropherograms) from the sequencer of choice.
• Automated alignments of partial sequences (incl. automated reverse complementation).
• Strip noise sequences at both ends of the sequenced fragment.
• Automated creation of a consensus sequence.
• Display of modified (proof-read) positions, amino acid translation.
• Jump to specific zones of interest (e.g. resistance encoding positions...).

One or more sequences can be imported and by using a procedure called "ProofRead", they are aligned with a single specific reference or with the most appropriate 16S reference automatically selected in a database. This database contains a subset of full length 16S references. A sequence consensus is obtained by comparing samples to the reference. An overview of the relative position of the samples and the reference can be displayed.

On demand, relevant positions for resistance can be denoted in the reference sequence by a colour code.

Single nucleotide fluorescent signal can be individually adjusted allowing a fine tuning of peak intensities (i.e. useful at the end of the sequence). Mismatches between consensus and reference sequence are clearly identified by highlighted positions. Ambiguities between samples sequences can be displayed. Nucleotides changes may be introduced. In case of correction of wrong nucleotides in the sample sequences, the changes are highlighted. Moreover, to facilitate the validation of numerous samples, the consensus is modifiable. In this case, any consensus modification leads to corresponding changes in the samples.

Sometimes, gaps or insertions are wrongly introduced in the sample sequences by sequencer softwares. Thus, they should be fixed by correcting sample sequences. A warning message appearing before saving will inform the user that such positions are still present.

A "realign function" allows the reassessment of the match-pairing between the samples and the reference. This can be performed after correction and validation steps. Another useful tool allows the retrieval of resistance relevant mutations (if defined), mismatches with reference, ambiguity between contigs and the highlight of special zones defined by the customer.

Garbage sequences may be automatically proposed and denoted by a colour. To trim regions encompassing garbage, select the last position before trimming in the nucleotide sequence, click to select upstream or downstream regions to be trimmed, respectively. More than one region could be selected at once.

Once the sequence is validated, the user should "Save" the sequence in IDNSTM. This will automatically store the validated sequence in the database. Nevertheless, the user can still revalidate the samples by using the ProofRead link in the sample sequence window. In this case, the same electropherograms files are automatically reloaded.

The presented experimental scheme shall serve to demonstrate and document in reproducible manner that the proposed sequences indeed fulfil the desired functions. Variations thereof are possible to the person skilled in the art without departing from the invention. The explicitly described protocol shall in any case not be interpreted to limit the scope of the invention as defined in the appended claims.

For verification of the broadband applicability of the proposed primers they have been tested experimentally on a huge number of genera and corresponding species. For all the following systems the primers have been found to work, i.e. to amplify the desired ranges efficiently. For each genus the corresponding species which were tested as well as the number of species are given in brackets. The total number of clinical isolates evaluated is 805 corresponding to ca. 190 species or yet undefined species (sp).

*Abiotrophia (adiacens 2, sp* 3), *Acetobacter(sp 1), Acinetobacter (baumannii 1, haemolyticus 2, lwoffii 3, sp 6), Actinobacillus (actinomycetemcomitans 2, sp* 2), *Actinomyces (birnadii 1, europae 1, israelii 3, meyeri 1, neuii 2, odontolyticus 1, radingae 1, sp 16, turicensis 1), Aerococcus (urinae 9), Aeromonas(hydrophila 1, sp 6, veronii 1), Alloiococcus (otitis 1), .Arcobacter (butzleri 1), Arthrobacter (oxydans 1, sp 1), Atopobium (rimae 1), Aureobacterium (sp 2), Bacillus (cereus 1, flexus 1, licheniformis 1, sp 6), Bifidobacterium (sp 4), Bordetella (sp 1), Brachybacterium (conglomeratum 1), Brevibacillus (sp 1). Brevibacterium (casei 1, sp 2), Brucella (sp1), Burkholderia (sp 3), Campylobacter (fetus 2, jejuni 9, sp 1), Capnocytophaga (canimorsus 5, sp 3), Cellulomonas (sp 1), Chryseobacterium (meningosepticum 1), Clostridium (botulinum 2, novyi 1, paraputrificum 1, septicum 1, sp 5, sporogenes 1, symbiosum 1, tertium 1), Corynebacterium (accolens 1, asperum 5, auris 1, macginleyi 2, otitidis 1. propinquum 1, seminale 3, sp 8, striatum 1, ulcerans 1, urealyticum 2, xerosis 2), Dermobacter (sp 1), Desulfovibrio (sp 1), Eikenella (corrodens 3, sp 1), Enterobacter (aerogenes 3, cloacae 2, sp 8), Enterococcus (avium 5, cecorum 1, durans 2, faecium 2, malodoratus 1, sp 11), Escherichia (coli 33), Fusobacterium (sp 2), Gardnerella (vaginalis 1), Gemella (haemolysans 1, sp 2),Gordona (sp 1), Haemophilus (aphrophilus 3, influenzae 4, paraphrophilus 2, sp 12), Helcococcus (sp 1), Kingella (sp 3). Klebsiella (pneumoniae 3, sp 2), Lactobacillus (acidophilus 1, casei 4, delbrueckii 3, gasseri 2, paracasei 1, pentosus 1, salivarius 1, sp 5, zeae 1), Legionella (micdadei 1). Listeria (ivanovii 1), Methylobacterium (sp 2). Micrococcus (sp 2), Moraxella (catarrhalis 5, nonliquefaciens 7, osloensis 3, phenylpyruvica 1, sp 4), Morganella (morganii 2), Mycobacterium (sp 1, alvei 2, aurum 1, avium 2, branderi 1, doricum 1, fortuitum 4, genavense 4, gilvum 3, gordonae-like 1, hassiacum 2, interjectum 3, kansasii 1, lenti,flavum 2, monacense 1, neoaurum 1, paratuberculosis 2, scrofulaceum 1, smegmatis 2, sp 53, szulgai 1, tusciae 3, hominis 1, pneumoniae 1), Neisseiria. (cinerea 1, meningitidis 206, sp 1), Nocardia (amycolata 1, asteroides 3, brasilieiisis 1, nova 2, sp 5), Ochrobactrum (anthropi 1), Oligella (urethralis 1), Pasteurella (canis 1. multocida 1), Pediococcus (acidilactici 1), Peptostreptococcus (anaerobius 1), Propionibacterium (acnes 3, propionicum 1), Proteus (mirabilis 12, penneri 2), Pseudomonas (aeruginosa 8, diminuta 3, sp 1), Ralstonia (sp 1), Rhodobacter (sp 1), Rhodococcus (sp 1), Ruminococcus (gnavus 1), Sanguibacter (suarezii 1),Sarcina (ventriculi 1), Shigella (boydii 1), Staphylococcus (aureus 6, capitis 1, epidermidis 4, haemolyticus 2, hominis 1, sp 2), Stenotrophomonas (maltophila 4), Streptobacillus (moniliformis 1), Streptococcus (anginosus 1, bovis 1, caprinus 1, dysgalactiae 1, gordonii 1, milleri 1, mitis 36, mitis*/*pneumoniae 4, mutans 1, parasanguis 2, pneumoniae 32, pyogenes 3, salivarius 3,sp 17), Tsukamurella (sp 1), Turicella (otitidis 2), Ureaplasma (urealyticum 2), variovorax (sp1).*

## Claims

1. Mixture of a pair of the distinct oligonucleotides for the qualitative and/or quantitative amplification using PCR and/or the sequencing of 16S rDNA-genes, wherein the oligonucleotides are selected from
an oligonucleotide consisting of the sequence
SEQ ID No. 1
and
an oligonucleotide consisting of the sequence
SEQ ID No. 2.

2. Mixture of a pair of oligonucleotides according to claim 1, wherein selective hybridisation takes place under stringent conditions only.

3. Mixture of a pair of oligonucleotides according to any of the preceding claims, wherein at least one of them comprises and/or is linked to and/or is attached to at least one functional element selected from: marker, carrier, antibody, capture molecule, bead, in particular magnetic bead.

4. Mixture of a pair of oligonucleotides according to any of the preceding claims, wherein at least one of them is attached to a matrix.

5. Mixture of the pair of the oligonucleotides according to any of the preceding claims for amplification using PCR, comprising these oligonucleotides in equal amounts.

6. Method for the specific amplification and/or the sequencing of 16S rDNA-genes, as well as of fragments thereof and RNA derived thereof, wherein as primer a mixture according to one of claims 1 - 5 is used.

7. Method according to claim 6, wherein for amplification of the 16S rDNA-gene, or the fragments thereof or the RNA derived thereof, the oligonucleotide is brought into contact with the mixture of the pair of the distinct oligonucleotides SEQ ID No. 1 and SEQ ID No. 2 acting as reverse and forward primer, respectively, and is subjected to a polymerase chain reaction leading to specific marker fragments with in the range of 100-2000, preferentially in the range of 800-1200 nucleotides.

8. Method according to claim 7, wherein contact between the 16S rDNA-gene, or the fragments thereof or the RNA derived thereof with the mixture of the pair of the distinct oligonucleotides SEQ ID No. 1 and SEQ ID No. 2 is established under stringent conditions.

9. Method according to claim 8, wherein under PCR using recombinant DNA polymerase from Thermus aquaticus the stringent conditions are defined as: 55°C, 500 nM MgCl₂.

10. Method according to any of claims 7-9, wherein in a first step the 16S rDNA-gene, or the fragments thereof or the RNA derived thereof is heat-denaturated in order to obtain single-stranded chains, subsequently these are brought into contact with a mixture of the pair of the distinct oligonucleotides SEQ ID No. 1 and SEQ ID No. 2, and then extended by means of a DNA polymerase, and wherein the heat-denaturation and the extension cycles are repeated in order to obtain a detectable amount of product.

11. Method according to claim 6, wherein for the sequencing using the chain termination method a sample is sequenced in both directions using either the reverse, SEQ ID No. 1, or the forward, SEQ ID No.2, as primer.

12. Method according to claim 11, wherein for the amplification steps stringent conditions are used.

13. Method according to claim 12 wherein in the amplification step under PCR using recombinant DNA polymerase from Thermus aquaticus the stringent conditions are defined as: 52°C, 500 nM MgCl₂.

14. Method for the identification of bacterial species based on their 16S rDNA-gene, wherein
a) sample material of a bacterial colony is isolated and the genetic material is extracted or at least made accessible to amplification;
b) the thus derived material is subjected to amplification in accordance with one of the claims 6-10;
c) the double-stranded DNA amplification products are purified;
d) the purified product is subjected to cycle-sequencing using a method in accordance with one of the claims 11-13;
e) the sequenced sample is purified;
f) the purified and sequenced sample is subjected to sequence electrophoresis and signal recording.

15. Method according to claim 14, wherein for the initial amplification in step b) as well as for the sequencing in step d) the same mixture of primers according to one of the claims 1-5 is used.

16. Method according to any of the claims 14 or 15, wherein for the identification of the bacterial genus/species/strains present in the bacterial colony the raw sequence data as obtained in step f) are automatically aligned, noise sequences are optionally stripped, a consensus sequence is created by comparing the measured data with sequence data from references from a database of known bacterial genus/species/strains.

## Patentansprüche

1. Mischung eines Paares unterschiedlicher Oligonukleotide für die qualitative und/oder quantitative Amplifikation durch PCR und/oder das Sequenzieren von 16S rDNA-Genen, wobei die Oligonukleotide aus
einem Oligonukleotid, bestehend aus der Sequenz
SEQ ID NO: 1 und
einem Oligonukleotid, bestehend aus der Sequenz
SEQ ID NO: 2
ausgewählt sind.

2. Mischung eines Oligonukleotidpaars nach Anspruch 1, wobei selektive Hybridisierung nur bei stringenten Bedingungen stattfindet.

3. Mischung eines Oligonukleotidpaars nach einem der vorhergehenden Ansprüche, wobei mindestens eines davon mindestens ein funktionelles Element, ausgewählt aus: Marker, Träger, Antikörper, Abfangmolekül, Kügelchen, insbesondere magnetisches Kügelchen, umfaßt und/oder damit verbunden ist und/oder darin befestigt ist.

4. Mischung eines Oligonukleotidpaars nach einem der vorhergehenden Ansprüche, wobei mindestens eines davon an eine Matrix gebunden ist.

5. Mischung des Oligonukleotidpaars nach einem der vorhergehenden Ansprüche zur Amplifikation durch PCR, umfassend diese Oligonukleotide in gleichen Mengen.

6. Verfahren für die spezifische Amplifikation und/oder die Sequenzierung von 16S rDNA-Genen, wie auch von Fragmenten davon und davon stammender RNA, wobei als Primer eine Mischung nach einem der Ansprüche 1 - 5 verwendet wird.

7. Verfahren nach Anspruch 6, wobei zur Amplifikation das 16S rDNA-Gen oder das Fragment davon oder die davon stammenden RNA mit der Mischung des Paars unterschiedlicher Oligonukleotide SEQ ID NO: 1 und SEQ ID NO: 2, die jeweils als Rückwärts- und Vorwärts-Primer dienen, in Kontakt gebracht wird und einer Polymerase-Kettenreaktion unterworfen wird, was zu spezifischen Markerfragmenten im Bereich von 100 bis 2000, vorzugsweise im Bereich von 800 bis 1200 Nukleotiden führt.

8. Verfahren nach Anspruch 7, wobei Kontakt zwischen dem 16S rDNA-Gen oder dem Fragment davon oder der davon stammenden RNA mit der Mischung des Paars unterschiedlicher Oligonukleotide der SEQ ID NO: 1 und SEQ ID NO: 2 unter stringenten Bedingungen hergestellt wird.

9. Verfahren nach Anspruch 8, wobei während der PCR unter Verwendung rekombinanter DNA-Polymerase aus *Thermus aquaticus* die stringenten Bedingungen definiert sind als: 55°C, 500 nM MgCl₂.

10. Verfahren nach einem der Ansprüche 7 bis 9, wobei in einem ersten Schritt das 16S rDNA-Gen oder die Fragmente davon oder die davon stammende RNA hitzedenaturiert wird, um einzelsträngige Ketten zu erhalten, diese werden nachfolgend mit einer Mischung des Paars unterschiedlicher Oligonukleotide SEQ ID NO: 1 und SEQ ID NO: 2 in Kontakt gebracht und dann mit Hilfe einer DNA-Polymerase verlängert, und wobei die Hitzedenaturierung und die Verlängerungszyklen wiederholt werden, um eine nachweisbare Menge des Produkts zu erhalten.

11. Verfahren nach Anspruch 6, wobei für die Sequenzierung unter Verwendung des Kettenabbruchverfahrens eine Probe in beiden Richtungen unter Verwendung von entweder dem Rückwärts-, SEQ ID NO: 1, oder dem Vorwärts-Primer, SEQ ID NO: 2, sequenziert wird.

12. Verfahren nach Anspruch 11, wobei für die Amplifikationsschritte stringente Bedingungen verwendet werden.

13. Verfahren nach Anspruch 12, wobei in dem Amplifikationsschritt während der PCR unter Verwendung rekombinanter DNA-Polymerase aus *Thermus aquaticus* die stringenten Bedingungen definiert sind als: 52°C, 500 nM MgCl₂.

14. Verfahren für die Identifizierung von bakteriellen Spezies, basierend auf deren 16S rDNA-Gen, wobei
a) Probenmaterial einer Bakterienkolonie isoliert wird und das genetische Material extrahiert oder zumindest für die Amplifizierung zugänglich gemacht wird,
b) das so erhaltene Material der Amplifikation in Übereinstimmung mit einem der Ansprüche 6 bis 10 unterworfen wird,
c) die doppelsträngigen DNA-Amplifikationsprodukte gereinigt werden,
d) das gereinigte Produkt einer Zyklussequenzierung unter Verwendung eines Verfahrens in Übereinstimmung mit einem der Ansprüche 11 bis 13 unterworfen wird,
e) die sequenzierte Probe gereinigt wird,
f) die gereinigte und sequenzierte Probe einer Sequenzelektrophorese und Signalaufzeichnung unterworfen wird.

15. Verfahren nach Anspruch 14, wobei für die anfängliche Amplifikation in Schritt b) wie auch für die Sequenzierung in Schritt d) dieselbe Mischung von Primern nach einem der Ansprüche 1 bis 5 verwendet wird.

16. Verfahren nach einem der Ansprüche 14 oder 15, wobei für die Identifizierung des/der bakteriellen Genus/Spezies/Stämme, die in der bakteriellen Kolonie vorhanden sind, die in Schritt f) erhaltenen rohen Sequenzdaten automatisch abgeglichen werden, Rauschsequenzen gegebenenfalls entfernt werden, eine Konsensussequenz durch Vergleichen der gemessenen Daten mit Sequenzdaten aus Referenzen aus einer Datenbank bekannter bakterieller Genus/Spezies/Stämme erstellt wird.

## Revendications

1. Mélange comprenant une paire d'oligonucléotides distincts destinés à être amplifiés de manière qualitative et/ou quantitative en utilisant une PCR et/ou le séquençage des gènes de l'ADNr 16S,
dans lequel les oligonucléotides sont choisis parmi un oligonucléotide consistant en la séquence
SEQ ID N° 1
et
un oligonucléotide consistant en la séquence SEQ ID N° 2.

2. Mélange comprenant une paire d'oligonucléotides selon la revendication 1, dans lequel une hybridation sélective n'a lieu que dans des conditions stringentes.

3. Mélange comprenant une paire d'oligonucléotides selon l'une quelconque des revendications précédentes, dans lequel au moins l'un d'entre eux comprend et/ou est lié et/ou est fixé à au moins un élément fonctionnel choisi parmi : un marqueur, un transporteur, un anticorps, une molécule de capture, une bille, en particulier une bille magnétique.

4. Mélange comprenant une paire d'oligonucléotides selon l'une quelconque des revendications précédentes, dans lequel au moins l'un d'entre eux est fixé à une matrice.

5. Mélange comprenant la paire d'oligonucléotides selon l'une quelconque des revendications précédentes, destiné à être amplifié en utilisant une PCR, comprenant ces oligonucléotides en quantités égales.

6. Procédé d'amplification et/ou de séquençage spécifique des gènes de l'ADNr 16S, ainsi que des fragments de ceux-ci et de l'ARN dérivé de ceux-ci, dans lequel est utilisé un mélange selon l'une quelconque des revendications 1 à 5 comme amorce.

7. Procédé selon la revendication 6, dans lequel, pour l'amplification du gène de l'ADNr 16S, ou des fragments de celui-ci ou de l'ARN dérivé de celui-ci, l'oligonucléotide est mis en contact avec le mélange comprenant la paire d'oligonucléotides distincts de SEQ ID N° 1 et de SEQ ID N° 2 agissant comme amorce antisens et sens, respectivement, et est soumis à une réaction en chaîne par polymérase menant à des fragments marqueurs spécifiques situés dans la plage allant de 100 à 2 000, de préférence dans la plage allant de 800 à 1 200 nucléotides.

8. Procédé selon la revendication 7, dans lequel le gène de l'ADNr 16S, ou les fragments de celui-ci ou l'ARN dérivé de celui-ci sont mis en contact avec le mélange comprenant la paire d'oligonucléotides distincts de SEQ ID N° 1 et de SEQ ID N° 2 dans des conditions stringentes.

9. Procédé selon la revendication 8, dans lequel les conditions stringentes sous PCR utilisant l'ADN polymérase recombiné provenant de *Thermus aquaticus* sont définies comme suit : 55 °C, 500 nM de MgCl₂.

10. Procédé selon l'une quelconque des revendications 7 à 9, dans lequel, lors d'une première étape, le gène de l'ADNr 16S, ou les fragments de celui-ci ou l'ARN dérivé de celui-ci sont dénaturés par la chaleur dans le but d'obtenir des chaînes monocaténaires, puis celles-ci sont mises en contact avec un mélange comprenant la paire d'oligonucléotides distincts de SEQ ID N° 1 et de SEQ ID N° 2, et puis allongées en utilisant une ADN polymérase, et dans lequel les cycles de dénaturation par la chaleur et d'extension sont répétés dans le but d'obtenir une quantité détectable de produit.

11. Procédé selon la revendication 6, dans lequel, pour le séquençage utilisant le procédé de terminaison de chaîne, un échantillon est séquencé dans les deux directions en utilisant soit l'amorce antisens, de SEQ ID N° 1, soit l'amorce sens, de SEQ ID N° 2, comme amorce.

12. Procédé selon la revendication 11, dans lequel, pour les étapes d'amplification, des conditions stringentes sont utilisées.

13. Procédé selon la revendication 12, dans lequel, pour l'étape d'amplification, les conditions stringentes sous PCR utilisant l'ADN polymérase recombiné provenant de *Thermus aquaticus* sont définies comme suit : 52 °C, 500 nM de MgCl₂.

14. Procédé d'identification d'espèces bactériennes sur la base de leur gène d'ADNr 16S, dans lequel :
a) une substance échantillon d'une colonie de bactéries est isolée et le matériel génétique est extrait ou au moins rendu accessible à des fins d'amplification ;
b) la substance ainsi dérivée est soumise à une réaction d'amplification selon l'une quelconque des revendications 6 à 10 ;
c) les produits d'amplification de l'ADN double-brin sont purifiés ;
d) le produit purifié est soumis à un séquençage cyclique en utilisant un procédé selon l'une quelconque des revendications 11 à 13 ;
e) l'échantillon séquencé est purifié ;
f) l'échantillon purifié et séquence est soumis à une électrophorèse des séquences et à l'enregistrement des signaux.

15. Procédé selon la revendication 14, dans lequel, lors de l'amplification initiale de l'étape b) ainsi que lors de l'étape de séquençage de l'étape d), le même mélange d'amorces selon l'une quelconque des revendications 1 à 5 est utilisé.

16. Procédé selon l'une quelconque des revendications 14 ou 15, dans lequel pour l'identification du genres / espèces / souches des bactéries présentes dans la colonie de bactéries, les données des séquences brutes telles qu'obtenues à l'étape f) sont alignées automatiquement, les séquences des bruits sont éventuellement retirées, une séquence fondamentale est créée en comparant les données mesurées aux données de séquence provenant de références appartenant à une base de données de bactéries de genres / espèces / souches connus.
